# EUROPEAN PATENT APPLICATION

(11) **EP 2 578 218 A1**
(43) Date of publication of application: **10.04.2013**
(21) Application number: 11184062.5
(22) Date of filing: 06.10.2011
(51) Int. Cl.: A61K 31/4422, A61P 31/16

(54) **Antiviral efficacy of disodium 2,6-dimethyl-1,4-dihydropyridine-3,5-bis(carbonyloxyacetate) and its derivatives**

(71) Applicant: Grindeks, A Joint Stock Company, 1057 Riga (LV)
(72) Inventor: Pubulis, Kaspars, LV-1057 Riga (LV); Stonans, Ilmars, LV-1057 Riga (LV); Jonane-Osa, Indra, LV-1057 Riga (LV); Jansone, Ilze, LV-1057 Riga (LV); Bisenieks, Egils, LV-1006 Riga (LV); Kalvins, Ivars, LV-1006 Riga (LV); Vigante, Brigita, LV-1006 Riga (LV); Bruvere, Imanta, LV-1006 Riga (LV); Uldrikis, Janis, LV-1006 Riga (LV); Zuka, Liga, LV-1006 Riga (LV); Poikans, Janis, LV-1006 Riga (LV); Neidere, Zaiga, LV-1006 Riga (LV)

(57) **Abstract**

Antiviral efficacy of disodium 2,6-dimethyl-1,4-dihydropyridine-3,5-bis(carbonyloxyacetate) and its derivatives (compound with general formula I) 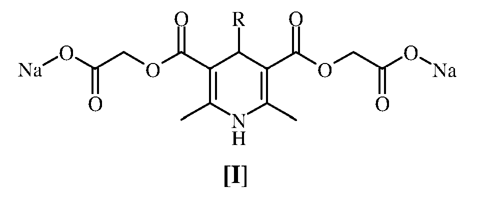 wherein R is hydrogen, methyl or ethyl.

## Description

### Technical Field

The present invention relates to antiviral efficacy of disodium 2,6-dimethyl-1,4-dihydropyridine-3,5-bis(carbonyloxyacetate) and its derivatives (compound with general formula I) 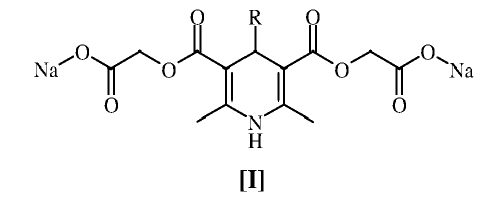 wherein R is hydrogen, methyl or ethyl

### Background Art

Influenza, commonly called "the flu," is an illness caused by RNA viruses that infect the respiratory tract of many animals, birds, and humans. In most people, the infection results in the person getting fever, cough, headache, and malaise (tired, no energy); some people also may develop a sore throat, nausea, vomiting, and diarrhea. The majority of individuals has symptoms for about one to two weeks and then recovers with no problems. However, compared with most other viral respiratory infections, such as the common cold, influenza (flu) infection can cause a more severe illness with a mortality rate (death rate) of about 0.1 % of people who are infected with the virus. Some influenza viruses develop resistance to the antiviral medicines, limiting the effectiveness of treatment.

There are known some medicaments which are effective against influenza viruses, such 1-methyl-2-phenylthiomethyl-3-carbethoxy-4-dimethylaminomethyl-5-oxybromoindole monohydrate hydrochloride (*is marketed under the trade name Arbido*/*®*), Oseltamivir (*is marketed by Roche under the trade name Tamifluo®*) and others.

Oseltamivir is indicated for the treatment and prevention of infections due to influenza A and B virus. Oseltamivir was disclosed in EP 0759917 B (GILEAD SCIENCES INC) 12.04.2000.

GB 2234510 A (NAUCHNO-ISSLEDOVATELSKY INSTITUT MEDITSINSKOI RADIOLOGII AKADEMII MEDITSINSKIKH NAUK SSSR) 06.02.1991 disclosed 1-methyl-2-phenylthiomethyl-3-carbethoxy-4-dimethylaminomethyl-5-oxybromoindole monohydrate hydrochloride as an active agent in a pharmaceutical preparation of antiviral, interferon inducing and immunomodulating action.

The disodium 2,6-dimethyl-1,4-dihydropyridine-3,5-bis(carbonyloxyacetate) with anti-arrhythmic action, was disclosed in DUBUR, et al. Anti-arrhythmic action of preparations of the dihydropyridine series. Farmakol. Toksikol.. 1983, vol.46, no.6, p.20-24.

### Disclosure of Invention

An object of the present invention is to provide compounds, possessing antiviral activity.

The above-mentioned object is attained by providing disodium 2,6-dimethyl-1,4-dihydropyridine-3,5-bis(carbonyloxyacetate) and its derivatives (compound with general formula I) 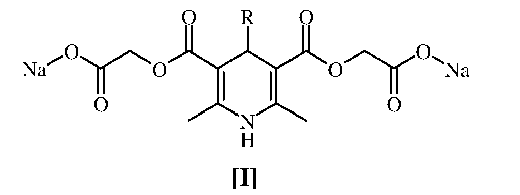 wherein R is hydrogen, methyl or ethyl
which, surprisingly and unexpectedly possessed antiviral efficacy.

The compounds according of general formula I are:
● disodium 2,6-dimethyl-1,4-dihydropyridine-3,5-bis(carbonyloxyacetate) having formula 11 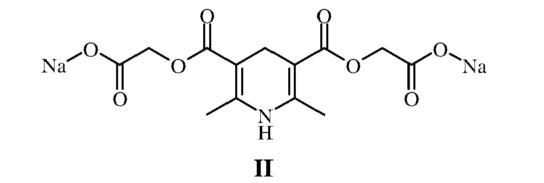
● disodium 2,4,6-trimethyl-1,4-dihydropyridine-3,5-bis-carbonyloxyacetate having formula III 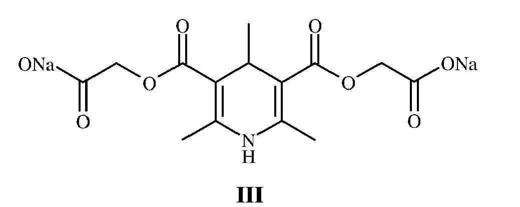
● disodium 4-ethyl-2,6-dimethyl-1,4-dihydropyridine-3,5-bis(carbonyloxyacetate) having formula IV

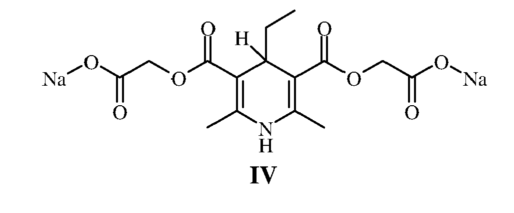

The compounds possess antiviral activity, due this properties disodium 2,6-dimethyl-1,4-dihydropyridine-3,5-bis(carbonyloxyacetate) and its derivatives - disodium 2,4,6-trimethyl-1,4-dihydropyridine-3,5-bis-carbonyloxyacetate and disodium 4-ethyl-2,6-dimethyl-1,4-dihydropyridine-3,5-bis(carbonyloxyacetate) (compounds with general formula I) may be used in medicine. The disodium 2,6-dimethyl-1,4-dihydropyridine-3,5-bis(carbonyloxyacetate) and its derivatives - disodium 2,4,6-trimethyl-1,4-dihydropyridine-3,5-bis-carbonyloxyacetate and disodium 4-ethyl-2,6-dimethyl-1,4-dihydropyridine-3,5-bis(carbonyloxyacetate) (compounds with general formula I) can be use as a solution of injection and as tablets or other solid dosage forms.

### Brief description of drawings

**FIG.1****.** represented antiviral efficacy of Oseltamivir on MDCK (*Madin-Darby Canine Kidney epithe*/*ial*) cell line *in vitro_{;}*
**FIG.2****.** represented antiviral efficacy of 1-methyl-2-phenylthiomethyl-3-carbethoxy-4-dimethylaminomethyl-5-oxybromoindole monohydrate hydrochloride on MDCK cell line *in vitro*;
**FIG.3****.** represented antiviral efficacy of disodium 2,6-dimethyl-1,4-dihydropyridine-3,5-bis(carbonyloxyacetate) on MDCK cell line *in vitro.*
**FIG.4****.** represented antiviral efficacy of disodium 2,4,6-trimethyl-1,4-dihydropyridine-3,5-bis-carbonyloxyacetate on MDCK cell line *in vitro.*
**FIG.5****.** represented antiviral efficacy of disodium 4-ethyl-2,6-dimethyl-1,4-dihydropyridine-3,5-bis(carbonyloxyacetate) on MDCK cell line *in vitro.*

The present invention will be described in more detail by referring to the following non-limiting examples.

### Best Mode for Carrying Out the Invention

For influenza virus, MDCK cells that were permissive of viral replication were grown up to sufficient numbers in growth media with supplements. Once MDCK cells were confluent they were seeded into 96 well flat-bottomed plates (2x10⁴ cells/well), incubated overnight and then infected with the influenza virus (H3N2) at the correct concentration and incubated in order to allow productive infection of the MDCK cells.

After the initial seeding of cells overnight (200pl/well), the medium was removed and influenza viral infection performed in a smaller volume (25µl/well) for 1 hour. After the 1 hour infection, the viral inoculum was removed and replaced with medium (200p1/well) containing test compound. Thus, disodium 2,6-dimethyl-1,4-dihydropyridine-3,5-bis(carbonyloxyacetate) and its derivatives - disodium 2,4,6-trimethyl-1,4-dihydropyridine-3,5-bis-carbonyloxyacetate and disodium 4-ethyl-2,6-dimethyl-1,4-dihydropyridine-3,5-bis(carbonyloxyacetate) (compounds with general formula I), were presented from 1 hour after viral infection until the end of the culture period.

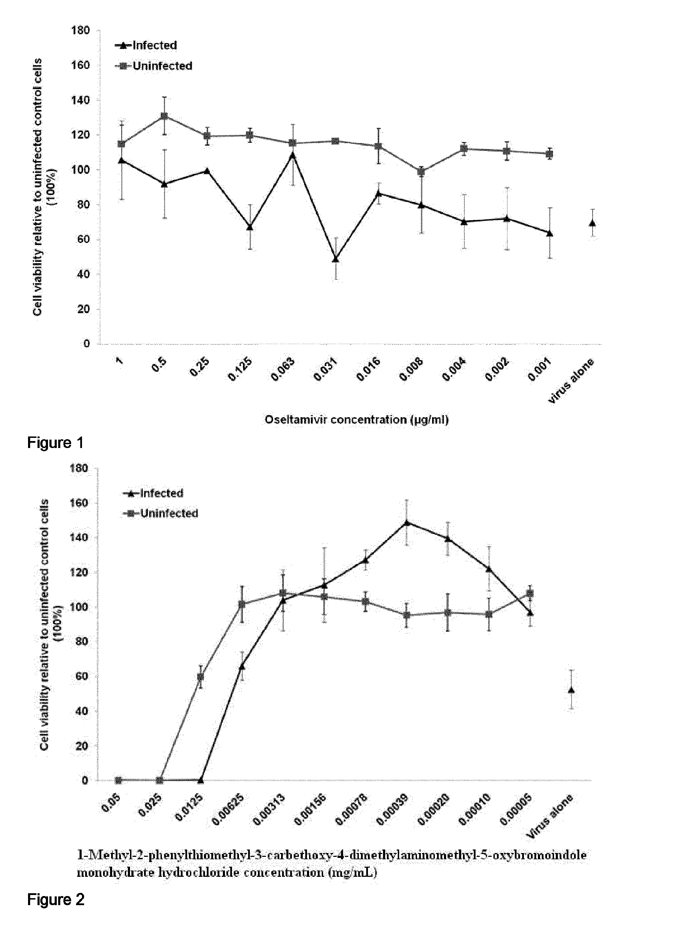

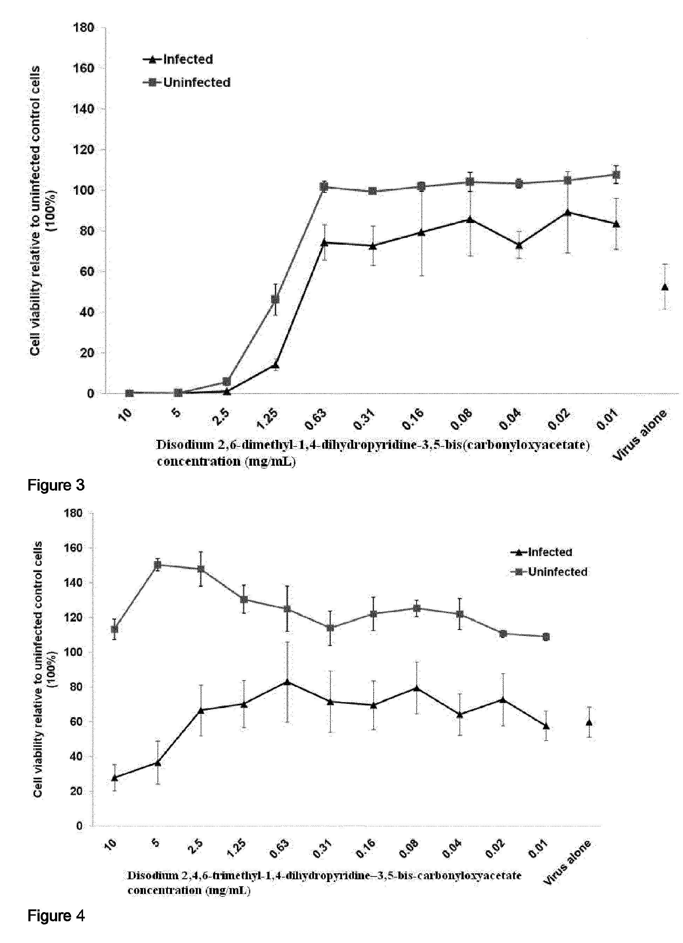

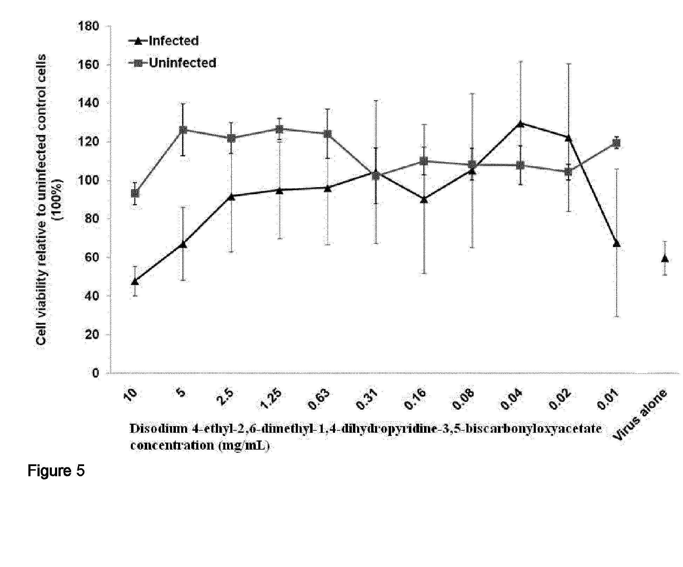

The antiviral efficacy of disodium 2,6-dimethyl-1,4-dihydropyridine-3,5-bis(carbonyloxyacetate) and its derivatives - disodium 2,4,6-trimethyl-1,4-dihydropyridine-3,5-bis(carbonyloxyacetate) and disodium 4-ethyl-2,6-dimethyl-1,4-dihydropyridine-3,5-bis(carbonyloxyacetate) (compounds with general formula I), were compared with already known medicaments, such as 1-methyl-2-phenylthiomethyl-3-carbethoxy-4-dimethylaminomethyl-5-oxybromoindole monohydrate hydrochloride and Oseltamivir, and results were represented in FIG.1., FIG.2., FIG.3., FIG.4. and FIG.5.

It was surprisingly and unexpectedly that disodium 2,6-dimethyl-1,4-dihydropyridine-3,5-bis(carbonyloxyacetate) and its derivatives - disodium 2,4,6-trimethyl-1,4-dihydropyridine-3,5-bis-carbonyloxyacetate and disodium 4-ethyl-2,6-dimethyl-1,4-dihydropyridine-3,5-bis(carbonyloxyacetate) (compounds with general formula I), are effective against influenza by using it over a wide concentration range, see FIG.3., FIG.4. and FIG.5.

## Claims

1. Use of 2,6-dimethyl-1,4-dihydropyridine-3,5-bis(carbonyloxyacetate) type compound, having general formula I 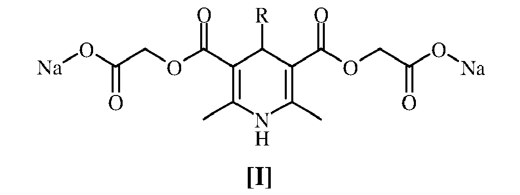 wherein R is hydrogen, methyl or ethyl, in the treatment of influenza.

2. Use according to claim 1, wherein 2,6-dimethyl-1,4-dihydropyridine-3,5-bis(carbonyloxyacetate) type compound is disodium 2,6-dimethyl-1,4-dihydropyridine-3,5-bis(carbonyloxyacetate).

3. Use according to claim 1, wherein 2,6-dimethyl-1,4-dihydropyridine-3,5-bis(carbonyloxyacetate) type compound is disodium 2,4,6-trimethyl-1,4-dihydropyridine-3,5-bis(carbonyloxyacetate).

4. Use according to claim 1, wherein 2,6-dimethyl-1,4-dihydropyridine-3,5-bis(carbonyloxyacetate) compound is disodium 4-ethyl-2,6-dimethyl-1,4-dihydropyridine-3,5-bis(carbonyloxyacetate).
